(19) Europäisches Patentamt
European Patent Office
Office européen des brevets

(11) Veröffentlichungsnummer: **0 370 481 B1**

(12) **EUROPÄISCHE PATENTSCHRIFT**

(45) Veröffentlichungstag der Patentschrift: **22.09.93**

(51) Int. Cl.⁵: **A61K 47/10**, A61K 47/12, A61K 31/545, A61K 31/43, A61K 31/71

(21) Anmeldenummer: **89121548.5**

(22) Anmeldetag: **21.11.89**

Die Akte enthält technische Angaben, die nach dem Eingang der Anmeldung eingereicht wurden und die nicht in dieser Patentschrift enthalten sind.

# (54) Pharmazeutische Präparate zur verbesserten Resorption antibakterieller Verbindungen.

(30) Priorität: **22.11.88 US 274590**

(43) Veröffentlichungstag der Anmeldung: **30.05.90 Patentblatt 90/22**

(45) Bekanntmachung des Hinweises auf die Patenterteilung: **22.09.93 Patentblatt 93/38**

(84) Benannte Vertragsstaaten: **AT BE CH DE FR GB IT LI LU NL SE**

(56) Entgegenhaltungen:
**EP-A- 0 024 031**
**EP-A- 0 108 295**
**EP-A- 0 126 348**
**EP-A- 0 152 896**
**EP-A- 0 179 583**

(73) Patentinhaber: **F. HOFFMANN-LA ROCHE AG**
**Postfach 3255**
**CH-4002 Basel(CH)**

(72) Erfinder: **Bachynsky, Maria Oksana**
**38 Carrie Court**
**Nutley, N.J. 07110(US)**
Erfinder: **Infeld, Martin Howard**
**6 Puers Placem**
**Upper Montclair, N.J. 07043(US)**
Erfinder: **Shah, Navnit**
**203 Beverly Hill Road**
**Clifton, N.J. 07012(US)**
Erfinder: **Unowsky, Joel**
**203 East McClellan Street**
**Livingston, N.J. 07039(US)**

(74) Vertreter: **Lederer, Franz, Dr. et al**
**Lederer, Keller & Riederer Patentanwälte**
**Lucile-Grahn-Strasse 22**
**D-81675 München (DE)**

EP 0 370 481 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft pharmazeutische Präparate, enthaltend (a) eine antibakteriell wirksame Verbindung und (b) eine wirksame Menge eines resorptionsverstärkenden 2-Komponenten-Systems bestehend aus (b1) Polyoxyethylenglykollauryläther mit 12 Ethylenoxideinheiten im Polyoxyethylenglykolteil (Laureth-12) und (b2) Natriumcaprylat. Gewünschtenfalls kann das erfindungsgemässe Präparat auch einen pharmazeutisch inerten Träger enthalten.

Das Dokument EP-A-0 126 348 beschreibt Formulierungen zur verbesserten rektalen Resorption von Arzneimitteln, die eine $C_{8-10}$-Fettsäure oder Salze davon und Polyoxyethylen (21) Lauryläther enthalten. Das Dokument EP-A-0 108 295 beschreibt Präparate zur enteralen Resorption von $\beta$-Lactamantibiotika, die als Resorptionsverstärker eine $C_{2-18}$-Fettsäure, ein $C_{2-12}$-Fettsäureglyzerid, ein mit einer $C_{2-12}$-Fettsäure voll oder partiell verestertes Propylenglykol, Polyethylenglykol oder Kohlehydrat oder Gemische davon enthalten. Das Dokument EP-A-0 152 896 beschreibt Suppositorien mit Ceftriaxon, die als Stabilisator $C_{2-18}$-Fettsäureglyzeride, $C_2$-$C_{18}$-Fettsäuren, mit einer $C_{2-18}$ Fettsäure voll oder partiell verestertes Propylenglykol, Polyethylenglykol oder Kohlehydrat, oder Gemische davon enthalten.

Es wurde gefunden, dass das oben bezeichnete resorptionsverstärkende System zu einer Erhöhung des Ausmasses der Resorption antibakterieller Verbindungen durch das Schleimhautgewebe und in die Blutbahn führt. Die Erfindung fördert somit die Resorption und gleichzeitig die Bioverfügbarkeit antibakterieller Verbindungen, die bei Verabreichung ohne den Resorptionsverstärker auf anderem Wege als parenteral nur schlecht oder überhaupt nicht in nennenswertem Mass resorbiert werden. Damit wird die Herstellung und die Verwendung einer grösseren Anzahl von Dosierungsformen für solche Verbindungen möglich gemacht. Die erfindungsgemäßen pharmazeutischen Präparate fördern auch die Resorption und Bioverfügbarkeit antibakterieller Verbindungen, die sonst durch Schleimhautgewebe nur mässig resorbiert werden und verstärken daher auch die Wirksamkeit solcher therapeutischer Verbindungen.

Die Erfindung betrifft pharmazeutische Kompositionen zur Verabreichung in jeder Dosierungsform, die für die orale oder rektale Verabreichung geeignet ist. Inbegriffen sind dabei orale und rektale Typen pharmazeutischer Präparate. die wirksame Mengen einer antibakteriellen Verbindung und des erfindungsgemässen Resorptionsverstärkungssystems mit oder ohne Anwesenheit inerter Träger und pharmazeutisch anwendbarer Hilfsmittel enthalten.

Die Ausdrücke "antibakteriell" und "antibiotisch" werden hier auswechselbar verwendet und bezeichnen bakterizide oder bakteriostatische Verbindungen, die als Stoffwechselprodukte eines Mikroorganismus, auf synthetischem Wege oder durch eine Kombination mikrobieller und chemischer Verfahren (semi-synthetisch) erhalten wurden.

Für die Anwendung in der vorliegenden Erfindung kommt jede antibiotische Substanz in Betracht, die zur Bekämpfung einer bakteriellen Infektion in einem Wirt geeignet ist, einschliesslich solcher Antibiotika, die nur mässig resorbiert werden, wenn sie nicht injiziert oder nicht infundiert werden. Das wichtigste Gebiet der Erfindung liegt in der Anwendung zur Verstärkung der Resorption und Bioverfügbarkeit von Antibiotika, die grösstenteils nur durch Injektion oder Infusion effektiv verabreicht werden können, weil sie mit anderen Verabreichungswegen nicht oder schlecht resorbiert werden.

Unter den bevorzugten antibakteriellen Verbindungen die als therapeutische Substanz erfindungsgemäss verwendet werden können sind $\beta$-Lactamantibiotika, insbesondere Verbindungen mit einem $\beta$-Lactamring als zentrale Struktur. d.h. mit dem Strukturelement

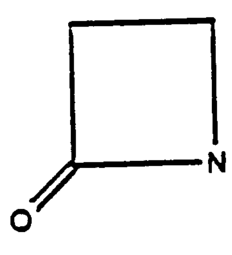

die in verschiedenen Stellungen des Rings substituiert sein können oder die mit anderen Ringen oder Ringsystemen, die für sich wiederum substituiert oder unsubstituiert sein können, kondensiert sein können. Beispiele solcher $\beta$-Lactamantibiotika sind Penicilline, Cephalosporine, Peneme, Carbapeneme und monocyclische $\beta$-Lactame.

Besonders bevorzugte β-Lactamantibiotika zur Anwendung der vorliegenden Erfindung sind Verbindungen der Formel

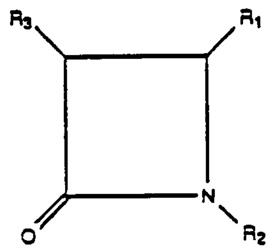

worin $R_1$ Wasserstoff, Alkyl oder substituiertes Alkyl, $R_2$ $SO_3^-M^+$, $M^+$ ein Proton oder ein Kation, $R_3$ eine Acylaminogruppe oder Hydroxyalkyl oder $R_1$ und $R_2$ zusammen mit dem β-Lactam (Azetidinon)-Ring, an die sie gebunden sind die Gruppe

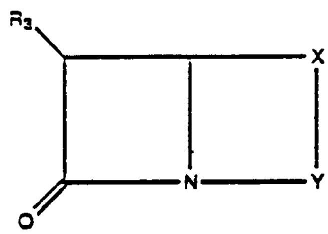

darstellen, worin X -S-, -O, -SO-, $-SO_2$, $-CH_2$ oder $-CH(CH_3)$ ist und Y eine Gruppe

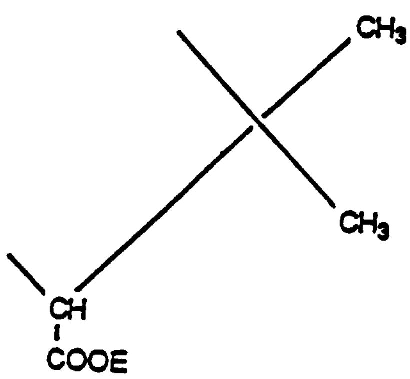

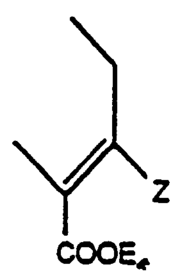

oder

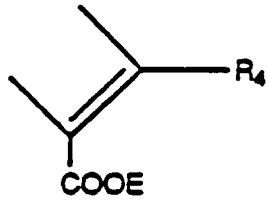

bedeutet, worin $R_4$ eine substituierte Thiogruppe, wie Aethylthio,

$-SCH_2CH_2NH_2$,

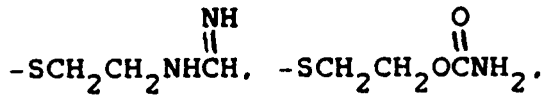

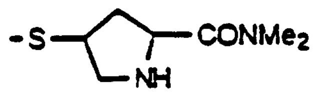

oder eine fakultativ substituierte niedere Alkylgruppe wie Aminomethyl, Acylaminomethyl,

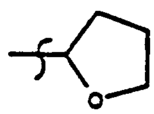

oder eine substituierte Oxygruppe wie Carbamoyloxy

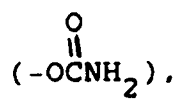

darstellt, das C-Atom, das die -COOE-Gruppe trägt, an das Stickstoffatom des β-Lactamrings gebunden ist, Z Wasserstoff, Halogen, Alkoxy oder $CH_2T$ ist, wobei T Wasserstoff, Alkyl, -CO-O-, Pyridinium, Carboxamidopyridinium, Aminopyridinium, Carbamoyloxy, Azido, Cyano, Hydroxyl, die Gruppe -S-Phenyl darstellt, die substituiert sein kann, oder die Gruppe -S-het darstellt, worin "het" ein fakultativ substituierter 5- oder 6-gliedriger heterocyclischer Ring ist und wobei E Wasserstoff, ein pharmazeutisch anwendbarer Estergruppe oder ein salzbildendes Kation darstellt.

Beispiele 5- oder 6-gliedriger heterocyclischer Ringe "het" sind die folgenden:

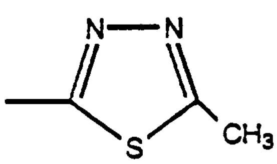

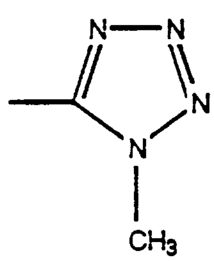

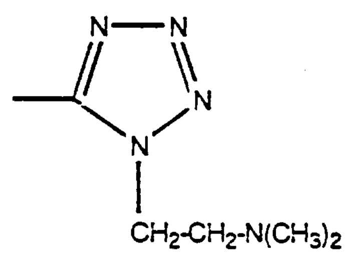

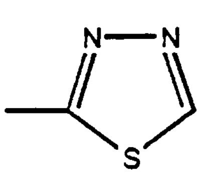

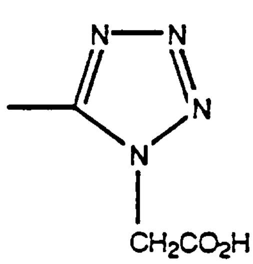

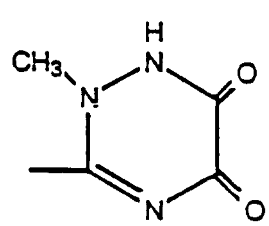

Besonders bevorzugte β-Lactamantibiotika und deren pharmazeutisch anwendbare Salze, Ester und Hydrate sind Ceftriaxon, ein Cephalosporin, das in der U.S. Patentschrift 4,327,210 beschrieben ist; Carumonam, ein monocyclisches β-Lactam, das in der Europäischen Patentschrift EP 73061 beschrieben ist; Piperacillin, ein Penicillin, das in der U.S. Patentschrift 4,112,090 beschrieben ist; Cefamandol, ein

Cephalosporin, das in der U.S. Patentschrift 3,641,021 beschrieben ist; Mezlocillin, ein Penicillin, das in der U.S. Patentschrift 3,974,142 beschrieben ist; und Cefazolin, ein Cephalosporin, das in der U.S. Patentschrift 3,516,997 beschrieben ist. Weitere Beispiele solcher Verbindungen sind Cefoxithin, Cefmetazol, Cefotetan, Moxalactam, Cefuroxim, Ceforamid, Cefoperazon, Ceftizoxim, Cefotaxim, Cefmenoxim, Ceftazidim, Cefsulodin, Cefazolin, Cephalexin, Azlocillin, Penicillin G, Temocillin, Sulbenicillin, Ticarcillin, Mecillinam, Amoxicillin, Methicillin, Carbenicillin, Thienamycin, N-Formimidoylthienomycin, Sulbactam und Azthreonam.

Ein weiterhin bevorzugtes $\beta$-Lactamantibiotikum zur Anwendung in der Erfindung ist die Verbindung (E)-2-(Isobutoxycarbonyl)-2-pentenyl(6R,7R)-7-[(Z) -2-(2-amino-4-thiazolyl)-2-(methoxyimino)acetamido] -3-(azidomethyl)-8-oxo-5--thia-1-azabicyclo[4.2.0]oct -2-ene-2-carboxylat, die in der Europäischen Patentpublikation A2-0318767 beschrieben ist.

Weiterhin umfasst die Erfindung die Anwendung auf Antibiotika, die keine $\beta$-Lactame sind, beispielsweise Vancomycin und Gentamicin, deren Resorption und Bioverfügbarkeit durch die Anwendung des erfindungsgemässen Resorptionsvertärkungssystems verbessert werden.

Das als Laureth-12 bezeichnete Material ist unter der Bezeichnung MACOL (R) LA-12 (Hersteller: Mazer Chemicals Company, Gurnee, Illinois) handelsüblich.

Die relativen Mengenverhältnisse der beiden Komponenten, die im erfindungsgemässen Resorptionsverstärkungssystem enthalten sind, können variiert werden, um optimale Resultate für eine bestimmte Ausführungsform der Erfindung zu erreichen. Vorzugsweise liegt das Gewichtsverhältnis von (b 1) zu (b 2) im Bereich von 1:50 bis 50:1, vorzugsweise von 1:10 bis 10:1 und äusserst bevorzugt von 1:4 bis 4:1.

Die wirksame Menge der Komponente (b) in dem erfindungsgemässen Präparat hängt von Faktoren wie der besonderen antibakteriellen Verbindung, die angewandt wird, und deren Menge ab, wie auch vom Alter des zu behandelnden Patienten.

Im allgemeinen ist es für orale Dosierungsformen bevorzugt, 50 bis 1000 mg, insbesondere 100 bis 500 mg des Resorptionsverstärkungssystems pro Dosiseinheit des pharmazeutischen Präparates anzuwenden. Diese Präparate enthalten üblicherweise die antibakterielle Verbindung in Mengen von 10 bis 500 mg, insbesondere von 50 bis 200 mg pro Dosiseinheit.

Rektale Dosierungsformen enthalten üblicherweise 50 bis 1500 mg, insbesondere 50 bis 600 mg des Resorptionsverstärkungssystems pro Dosiseinheit. Solche Präparate enthalten die antibakterielle Verbindung üblicherweise in Mengen von 10 bis 3000 mg, insbesondere von 100 bis 1500 mg pro Dosiseinheit.

Der Ausdruck "Dosiseinheit" wird hier im üblichen Sinne gebraucht, um eine einzelne Darreichung des Medikamentes in der angegebenen Menge zu bezeichnen. Diese Menge kann in Form einer einzelnen Pille, Tablette, Kapsel oder Suppositorium verabreicht werden oder alternativ in Vielfachen von zwei oder mehr solcher Dosiseinheiten, die dann zusammen die angegebene Menge des Wirkstoffs enthalten.

Die beschriebene antibakterielle Verbindung (a) und die Komponenten (b1) und (b2) des Resorptionsverstärkungssystems können in einen Träger eingearbeitet werden, falls dies gewünscht ist. Als Träger kann jeder pharmazeutisch anwendbare Feststoff, Halbfeststoff oder flüssige Träger, in dem diese Komponenten löslich oder leicht verteilbar sind, angewandt werden. Beispiele dafür sind Kakaobutter, Polyethylenglykole, Polypropylenglykole, Methylcellulose, Carboxymethylcellulose und semi-synthetische Basen, wie Suppocire® (Gattefosse Corp., Paris). Vorzugsweise ist das Vehikel ein Feststoff. Bevorzugt als Feststoff für die erfindungsgemässe Anwendung sind Gemische von Mono-, Di- und Triglyzeriden von $C_{12}$-$C_{18}$ natürlichen gesättigten Fettsäuren, vorzugsweise Fettsäuren, die eine gerade Anzahl von C-Atomen haben ($C_{12}$, $C_{14}$, $C_{16}$). Besonders geeignet und bevorzugt sind pharmazeutische Basen von Dynamit Nobel unter der Handelsbezeichnung "WITEPSOL®".

Andere pharmazeutisch verträgliche Trägermaterialien können gewünschtenfalls je nach den besonderen Bedürfnissen angewandt werden. Ihre Auswahl liegt im Bereich des Fachwissens.

Falls ein Trägerstoff angewandt wird, liegen seine Mengen in Bereichen, die für pharmazeutische Trägermaterialien üblicherweise angewandt werden und die sicher verabreicht werden können.

Für die orale Verabreichung der erfindungsgemäsen Präparate ist eine magensaftresistent überzogene Formulierung bevorzugt, vorzugsweise eine solche in fester Form. Die Formulierung kann in Hartgelatine- oder Weichgelatinekapseln oder, falls sie flüssig ist, auf einem geeigneten Träger absorbiert sein unter Bildung eines freifliessenen Pulvers und dann in die Kapsel abgefüllt oder alternativ zu Pillen oder Tabletten verpresst sein. Andere Dosierungsformen sind Mikrokapseln oder Beadlets, bestehend aus der antibakteriellen Verbindung und dem Resorptionsverstärkungssystem, die in eine mit einem magensaftresistenten Ueberzug versehenen Kapsel eingehüllt sind.

Die Verwendung von magensaftresistenten Ueberzügen dient dazu, die antibakterielle Verbindung vor der Magenflüssigkeit zu schützen und einen optimalen Transport der antibakteriellen Verbindung zusammen mit dem Resorptionsverstärkungssystem in den Darmtrakt zu erreichen. Der magensaftresistente Ueberzug ist zum grössten Teil gegenüber der Magenflüssigkeit widerstandsfähig und bleibt unangegriffen, löst sich

aber in der Darmflüssigkeit, wobei der Wirkstoff freigesetzt wird.

Die Wirksamkeit eines besonderen magensaftresistenten Ueberzugsmaterials kann mit bekannten Verfahren gemessen werden. Beispielsweise sind geeignete magensaftresistente Ueberzugsmaterialien zur Anwendung in der vorliegenden Erfindung die folgenden:

Celluloseacetatphthalat
Celluloseacetattrimellitat
Hydroxypropylmethylcellulosephthalat
Hydroxypropylmethylcellulosephthalatsuccinat
Polyvinylacetatphthalat
Methacrylsäure
Methacrylsäureester

Diese Materialien können mit oder ohne Weichmacher, wie acetylierten Glyzeriden oder Diethylphthalat in an sich bekannter Weise angewandt werden.

Der Prozentsatz des angewandten Ueberzuges liegt üblicherweise zwischen 1 und 10 Gewichtsprozenten oder mehr, insbesondere zwischen 2 bis 8 Gewichtsprozent bezogen auf das Gesamtgewicht der Dosiseinheitsform, d.h. der gesamten Kapsel oder dem gesamten Tablettengewicht. Beispiele geeigneter Ueberzugsformulierungen sind nachstehend angegeben.

<u>Formulierungen für magensaftresistente Ueberzüge</u>

| Inhaltsstoff | Gewichtsprozent |
|---|---|
| <u>Präparat A</u>: | |
| Hydroxypropylmethylcellulosephthalat (HPMCP) | 5.0 |
| Triacetin | 0.5 |
| Methylenchlorid | 47.25 |
| Denaturierter Alkohol | 47.25 |

Präparat B:

| | |
|---|---|
| HPMCP | 10.0 |
| Titandioxid | 0.2 |
| Dimethylpolysiloxan | 0.05 |
| Aceton | 44.875 |
| Denaturierter Alkohol | 44.875 |

Präparat C:

| | |
|---|---|
| Celluloseacetatphthalat (CAP) | 8.5 |
| Diethylphalat | 1.5 |
| Titandioxid | 0.2 |
| Aceton | 44.9 |
| Denaturierter Alkohol | 44.9 |

Präparat D:

| | |
|---|---|
| Polyvinylacetatphthalat | 5.0 |
| Acetylierte Glyzeride | 0.8 |
| Methylenchlorid | 47.1 |
| Denaturierter Alkohol | 47.1 |

Präparat E:

| | |
|---|---|
| Methacrylsäure oder Methacrylsäure-ester (Eudragit® S or L, Rohm Pharma, GMBH, Wetterstadt, BRD) | 8.0 |
| Aceton | 46.0 |
| Absoluter Alkohol | 46.0 |
| Weichmacher | q.s. |

Die erfindungsgemäsen oralen Formulierungen können auch zusätzlich übliche Zusätze oder Hilfsstoffe enthalten in für solche Materialien üblichen Mengen. Beispielsweise können solche Zusätze Verdickungsmittel sein, wie Kieselsäure (beispielsweise Aerosilprodukte), Bentonite, colloidaler Ton, Carboxymethylcellulose, modifizierte Montmorillonite wie Alkylammoniumsalze von Montmorilloniten (beispielsweise Handelsprodukte wie Bentone®, organische Verdickungsmittel und Strukturbildner wie gesättigte höhere Fettsäuren und Alkohole mit 12-20 C-Atomen (beispielsweise Stearin- und Palmitinsäure oder Stearin und Cetylalkohol), Wachse, Monoglyzeride, gesättigter oder ungesättigter höherer Fettsauren, wie Stearinsäure, Palmitinsäure oder Oelsäure, Geliermittel wie Aluminiumstearat, Dispergierungsmittel, wie ionische, nicht-ionische

oder kationische oberflächenaktive Stoffe, Emulgatoren, wie Lezithin.

Die erfindungsgemässen Kompositionen können auch pharmazeutisch anwendbare Hilfsmittel wie Bindemittel oder Schmiermittel zum Tablettieren, Stabilisatoren, Antioxidantien, Fliessmittel (zur Verbesserung der Giessfähigkeit oder Fliessfähigkeit während der Verarbeitung), Konservierungsmittel, Geschmacksstoffe, Farbstoffe und Puffer enthalten. Alle diese Stoffe können aus Materialien ausgewählt werden, die für solche Anwendungszwecke bekannt sind.

Die verbesserte Resorption von Antibiotika durch Schleimhautgewebe mittels der erfindungsgemäsen Präparate wurde in in vivo-Tests geprüft.

Enterale Resorption in vivo (Ratten)

Weibliche Ratten im Gewicht von etwa 250 g wurden über Nacht fasten gelassen und mit Metofan® anästhesiert. Die Antibiotika wurden in Lösung mit oder ohne Resorptionsverstärker nach Bauchschnitt in das Duodenum unterhalb des Magenausgangs injiziert. Für Vergleichszwecke wurde die Lösung alternativ intravenös in die Schwanzvene appliziert.

Plasmaspiegel des Antibiotikums bei Ratten

Die Konzentration des Antibiotikums im Plasma der Ratte wurde nach verschiedenen Zeitintervallen nach intravenöser oder enteraler Verabreichung bestimmt. Blutproben wurden aus der Schwanzvene des Versuchstiers vor der Verabreichung des Antibiotikums und 5, 10, 20, 40, 60, 120, 240 und 360 Minuten danach entnommen, danach zentrifugiert und das Plasma bis zur Analyse eingefroren.

Bioassay der Plasmaproben

Die meisten der getesteten Antibiotika zeigten einen gewissen Grad von Proteinbindung, wenn das Antibiotika-haltige Plasma gegen das Antibiotikum in Wasser getestet wurde. Antibiotika, die nicht an Plasma gebunden waren, wurden mit Wasser verdünnt und gegen in Wasser hergestellte Standards geprüft. Für gebundene Antibiotika wurde der Einfluss der Proteinbindung eliminiert durch Verdünnen aller Standardlösungen und Proben im gesammelten Plasma. Im Fall von Ceftriaxon und Cefazolin wurde der Effekt der Bindung dadurch berücksichtigt, dass Plasmaproben mit Acetonitril mit einem Verdünnungsfaktor 1:12 deproteinisiert und gegen eine Standardkurve in Wasser verdünnt gemessen wurden. Antibiotikaspiegel wurden wie unten angegeben auf Agarplatten ermittelt.

| Antibiotikum | Testorganismus | Bereich der Standardkurven (µg/ml) | Testmedium | Volumen (µl) |
|---|---|---|---|---|
| Carumonam | E. coli. 1346 | 32-1 | AA#1[2] | 20 |
| Ampicillin[1] | M. lutea ATCC 9341 | 8-0.25 | AA#1 | 20 |
| Cefamandole[1] | M. lutea ATCC 9341 | 32-1 | AA#1 | 20 |
| Cefotaxime[1] | E. coli. 1346 | 8-0.25 or 16-0.5 | AA#1 | 20 |
| Cefoxitin[1] | S. aureus MB2786 | 64-4 | BHI[3] | 20 |
| Ceftriaxone[1] | E. coli. 1346 | 4-0.125 | AA#1 | 20 |
| Cefazolin[1] | S. aureus ATCC 25923 | 32-1 | AA#1 | 50 |
| | B. subtilis spores | 32-2 | AA#1 | 50 |
| Moxalactam[1] | E. coli. 1346 | 50-1.56 | AA#1 | 50 |
| Penicillin G[1] | M. lutea ATCC 9341 | 8-0.5 | AA#1 | 20 |
| Mezlocillin[1] | M. lutea ATCC 9341 | 16-1 | AA#1 | 50 |
| Gentamicin[1] | K. pneumoniae A | 80-2.5 | MH[4] | 50 |
| Vancomycin[1] | B. cereus ATCC 11778 | 64-2 | AA#8[5] | 50 |

[1] Antibiotika mit Proteinbindung im Rattenplasma.
[2] Antibiotischer Agar Nr. 1 (Difco).
[3] BHI = Brain Heart Infusion Media (Difco).
[4] MH = Mueller Hinton Agar (Difco).
[5] Antibiotischer Agar Nr. 8 (Difco).

Die Platten wurden bei 37°C über Nacht bebrütet und die Hemmzonen auf die nächsten 0,1 ml abgelesen. Die Berechnungen wurden mittels einer Autoassaymaschine (Giles Scientific, Inc., New York) vorgenommen. Vergleiche J.V. Bennett et al., Applied Microbiology 14, 170-177 (1966).

Die Resultate waren wie folgt:

Tabelle 1

| Enterale Resorption bei Ratten mit und ohne Resorptionsverstärker Dosis = 5 mg/0,5 ml | | |
|---|---|---|
| Antibiotika | Cmax (µg/ml | |
| | Kontrolle Wasser | Natriumcaprylat (30%) + Laureth-12* |
| Carumonam | 0.0 ± 0.0 | 6.3 ± 2.4 |
| Cefamandole | 1.3 ± 2.5 | 18.1 ± 3.7 |
| Cefazolin | 0.0 ± 0.0 | 40.7 ± 7.2 |
| Cefoxitin | 0.0 ± 0.0 | 16.2 ± 4.6 |
| Cefotetan | 0.0 ± 0.0 | 26.1 ± 11.4 |
| Gentamicin | 3.9 | 14.1 ± 6.9 |
| Mezlocillin | 0.0 ± 0.0 | 6.7 ± 1.7 |
| Moxalactam | 0.0 ± 0.0 | 19.9 ± 4.5 |
| Penicillin G | 0.5 ± 0.) | 7.4 ± 2.4 |
| Vancomycin | 2.9 ± 0.6 | 9.8 ± 3.5 |
| Ceftriaxone | 2.4 ± 1.9 | 53.7 ± 13.3 |

*Gewichtsverhältnis der anderen Komponente des Resorptionsverstärkers zu Laureth-12 war 8:1

Orale Resorption in vivo (Affen)

Erwachsene Affen (Papio anubis und Papio hamadryas) im Gewicht von 12-30 kg wurden über Nacht fasten gelassen, dann durch i.m.-Injektion von Ketaminhydrochlorid vor der Verabreichung des Antiobiotikums sediert. Jeder Affe erhielt durch Schlundsonde vier Hartgelatinekapseln mit jeweils 300 mg Ceftriaxon Natrium, 200 ml Natriumcaprylat, 75 mg Laureth-12 und 415 mg Witespol (R) H15. Die Bioverfügbarkeit dieser Formulierung betrug 15,7 ± 9.9 % und 15,0-66,3 µg/ml Cmax-Bereich, verglichen mit 0 % Bioverfügbarkeit und 0 µg/ml Cmax für den Kontrollwert (Ceftriaxon Natrium 300 mg, in der gleichen kapsel, ohne Resorptionsverstärker).

Orale Resorption in vivo (Hunde)

Für diesen Versuch wurden männliche Beagle-Hunde im Gewicht von 10-14 kg verwendet, die 2-3 Hartgelatinekapseln durch die Schlundsonde erhielten. Die Kapseln enthielten 300 mg Cetriaxon Natrium, 200 mg Natriumcaprylat, 75 mg Laureth-12 und 415 mg Witepsol (R) H15. Die Bioverfügbarkeit betrug 22,4 ± 13,5 %, Cmax war 14,4 ± 8,4 µg/ml, verglichen mit 0 % und 0 µg/ml für den Kontrollwert (Ceftriaxon Natrium, in der gleichen kapsel, ohne Resorptionsverstärker).

Rektale Resorption in vivo (Affen)

Für diesen Versuch wurden männliche und weibliche erwachsene Affen (Papio anubis und Papio hamadryas) im Gewicht vom 12-27 kg verwendet, die 24 Stunden vor der Verabreichung des Antibiotikums fasten gelassen wurden, dann durch i.m.-Injektionen von Ketamin-hydrochlorid vor der Verabreichung des Antibiotikums sediert wurden. Aus den nachstehend beschriebenen Formulierungen wurden Suppositorien hergestellt, die den Tieren verabreicht wurden. Die Rektalöffnungen wurden dann mit Klebeband verschlossen, um ein Herausdrücken der Suppositorienmasse zu vermeiden.

| | |
|---|---|
| Ceftriaxon-Na | 600 mg |
| Laureth-12 | 125 mg |
| Na-caprylat | 200 mg |
| Witepsol® H15 | 1075 mg |
| | Total: 2000 mg |

Zur Messung der Resorption des Antibiotikums in den Blutstrom wurden Blutproben aus der Femoralregion vor der Verabreichung des Antibiotikums und 15, 30, 60, 120, 240, 360 und 480 Minuten danach entnommen. Proben wurden zentrifugiert und wie oben beschrieben vermessen.

Die Bioverfügbarkeit betrug 49,3 ± 13.7 %, der Cmax-Bereich war 68,1-1002.8 µg/ml, verglichen mit 4,3 % Bioverfügbarkeit und 0,3-9.0 µg/ml Cmax-Bereich für den Kontrollwert (600 mg Ceftriaxon Natrium in Suppositoriummasse ohne Resorptionsverstärker).

Nachstehend sind einige Formulierungen für verschiedene Dosierungsbereiche für die erfindungsgemässen Präparate angeführt. Obschon in diesen Beispielen Ceftriaxon, das bevorzugte Antibiotikum im Rahmen der Erfindung, in den Beispielen figuriert, können auch andere Antibiotika in passenden Mengen eingesetzt werden.

| ORALE FORMULIERUNGEN | | | | |
|---|---|---|---|---|
| Ceftriaxon (Natrium) | 60 mg | 120 mg | 210 mg | 300 mg |
| Natriumcaprylat | 200 mg | 200 mg | 200 mg | 200 mg |
| Laureth-12 | 75 mg | 75 mg | 75 mg | 75 mg |
| Witepso® H15 | 365 mg | 365 mg | 365 mg | 365 mg |

| REKTALE FORMULIERUNGEN | | | | |
|---|---|---|---|---|
| Ceftriaxon (Natrium) | 180 mg | 300 mg | 600 mg | 1200 mg |
| Natriumcaprylat | 200 mg | 200 mg | 200 mg | 400 mg |
| Laureth-12 | 125 mg | 125 mg | 125 mg | 250 mg |
| Witepso® H15 | 1495 mg | 1375 mg | 1075 mg | 2150 mg |

Die obigen Formulierungen können wie folgt hergestellt werden:

Orale Formulierungen

Die Base (Witepsol® H15) wird auf 55 °C erwärmt und die Komponenten des Resorptionsverstärkersystems der Schmelze zugemischt. Die Schmelze wird dann auf 45 °C gekühlt und der Wirkstoff (Ceftriaxon Natrium) der geschmolzenen Masse, zugesetzt und gleichmässig darin verteilt. Die Masse wird homogenisiert bis eine einheitliche Suspension erhalten wird. Diese wird dann in Gelatinekapseln abgefüllt, gegebenenfalls versiegelt und die Kapseln mit einem magensaftresistenten Ueberzug versehen.

Rektale Formulierungen

Die Base (Witepsol® H15) wird auf 55 °C erwärmt und die Komponenten des Resorptionsverstärkungssystems der Schmelze zugemischt. Die Schmelze wird dann auf 45 °C gekühlt und der Wirkstoff (Ceftriaxon Natrium) der geschmolzenen Masse zugesetzt und gleichmässig darin verteilt. Die Masse wird homogenisiert, bis eine einheitliche Suspension erhalten wird, dann in Suppositorienschalen gefüllt und aushärten gelassen.

**Patentansprüche**

**1.** Pharmazeutisches Präparat, enthaltend (a) eine antibakteriell wirksame Verbindung, und (b) eine wirksame Menge eines resorptionsverstärkenden 2-Komponenten-systems, bestehend aus (b 1) Polyoxyethylenglykol-laurylläther mit 12 Ethylenoxideinheiten im Polyoxyethylenglykolteil (Laureth 12) und (b 2) Natriumcaprylat.

**2.** Präparat nach Anspruch 1 , worin die antibakteriell wirksame Verbindung ein $\beta$-Lactam ist.

**3.** Präparat nach Anspruch 2, worin das $\beta$-Lactam die Formel

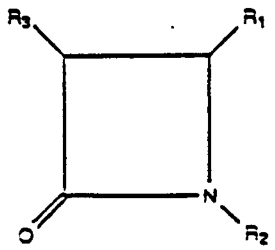

besitzt,
worin $R_1$ Wasserstoff, Alkyl oder substituiertes Alkyl, $R_2$ $SO_3$-M+, M+ ein Proton oder ein Kation, $R_3$ eine Acylaminogruppe oder Hydroxyalkyl oder $R_1$ und $R_2$ zusammen mit dem $\beta$-Lactam (Azetidinon)-Ring, an die sie gebunden sind die Gruppe

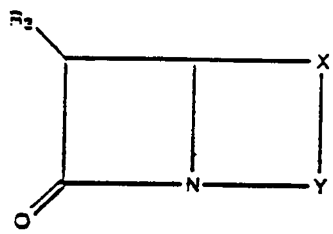

darstellen, worin X -S-, -O, -SO-, $-SO_2$, $-CH_2$ oder $-CH(CH_3)$ ist und Y eine Gruppe

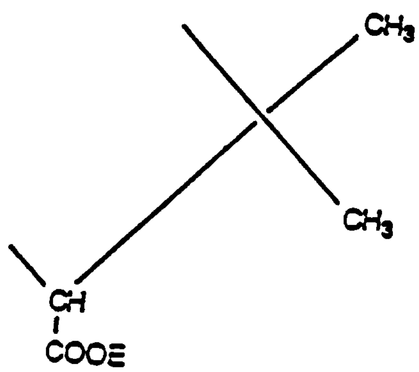

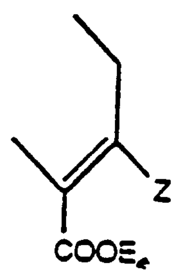

oder

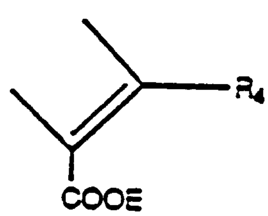

bedeutet, worin $R_4$ eine substituierte Thiogruppe, wie Aethylthio,

$-SCH_2CH_2NH_2$,

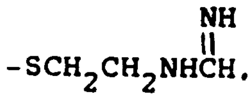 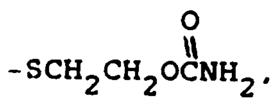 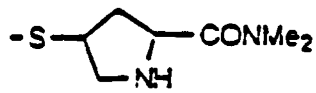

oder eine fakultativ substituierte niedere Alkylgruppe wie Aminomethyl, Acylaminomethyl,

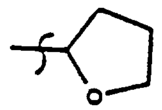

oder eine substituierte Oxygruppe wie Carbamoyloxy

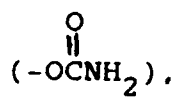

darstellt, das C-Atom, das die -COOE-Gruppe trägt, an das Stickstoffatom des $\beta$-Lactamrings gebunden ist, Z Wasserstoff, Halogen, Alkoxy oder $CH_2T$ ist, wobei T Wasserstoff, Alkyl, -CO-O-, Pyridinium, Carboxamidopyridinium, Aminopyridinium, Carbamoyloxy, Azido, Cyano, Hydroxyl, die Gruppe -S-Phenyl darstellt, die substituiert sein kann, oder die Gruppe -S-het darstellt, worin "het" ein fakultativ substituierter 5- oder 6-gliedriger heterocyclischer Ring ist und wobei E Wasserstoff, ein pharmazeutisch anwendbarer Estergruppe oder ein salzbildendes Kation darstellt.

**4.** Präparat nach Anspruch 2 , worin die antibakteriell wirksame Verbindung (E)-2-(Isobutoxycarbonyl)-2-pentenyl-(6R,7R) -7-[(Z)-2-(2-amino-4-thiazolyl)-2-(methoxyimino)-acetamido] -3-(azidomethyl)-8-oxo-5-thia-1-azabicyclo-[4.2.0]oct -2-en-2-carboxylat ist.

**5.** Präparat nach Anspruch 2 , worin die antibakteriell wirksame Verbindung Ceftriaxon oder ein pharmazeutisch anwendbares Salz, Ester oder Hydrat davon ist.

**6.** Präparat nach den Ansprüchen 1-5 in einer magensaftresistent überzogenen, oralen Darreichungsform.

**7.** Präparat nach den Ansprüchen 1-5 in einer rektalen Darreichungsform.

## Claims

**1.** A pharmaceutical preparation containing (a) an antibacterial compound and (b) an effective amount of an absorption enhancing 2-component system consisting of (b1) polyoxyethylene glycol lauryl ether with 12 ethylene oxide units in the polyoxyethylene glycol part (Laureth-12) and (b2) sodium caprylate.

**2.** A preparation according to claim 1, in which the antibacterial compound is a $\beta$-lactam.

**3.** A preparation according to claim 2, in which the $\beta$-lactam has the formula

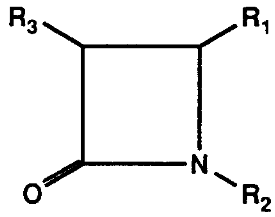

in which $R_1$ represents hydrogen, alkyl or substituted alkyl, $R_2$ represents $SO_3$-M+, M+ represents a proton or a cation, $R_3$ represents an acylamino group or hydroxyalkyl or $R_1$ and $R_2$ together with the $\beta$-lactam (azetidinone) ring to which they are bonded represent the group

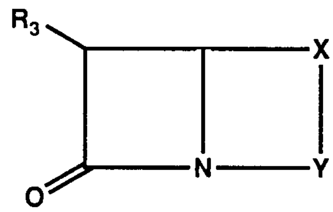

in which X is -S-, -O-, -SO-, $-SO_2$, $-CH_2$ or $-CH(CH_3)$ and Y signifies a group

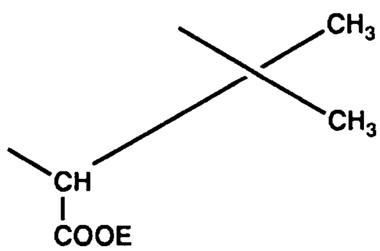

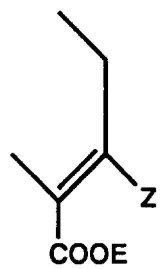

or

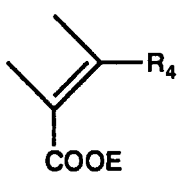

in which $R_4$ represents a substituted thio group such as ethylthio,
$-SCH_2CH_2NH_2$,

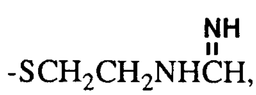

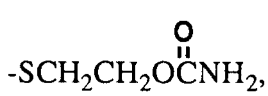

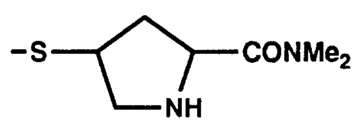

or an optionally substituted lower alkyl group such as aminomethyl, acylaminomethyl,

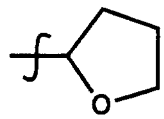

or a substituted oxy group such as carbamoyloxy

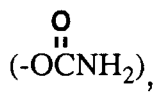

the C atom which carries the -COOE group is bonded to the nitrogen atom of the $\beta$-lactam ring, Z is hydrogen, halogen, alkoxy or $CH_2T$, with T denoting hydrogen, alkyl, -CO-O-, pyridinium, carboxamidopyridinium, aminopyridinium, carbamoyloxy, azido, cyano, hydroxyl, the group -S-phenyl, which can be substituted, or the group -S-het, wherein "het" is an optionally subsituted 5- or 6-membered heterocyclic ring, and E represents hydrogen, a pharmaceutically usable ester group or a salt-forming cation.

**4.** A preparation according to claim 2, in which the antibacterial compound is (E)-2-(isobutoxycarbonyl)-2-pentenyl-(6R,7R)-7-[(Z)-2-(2-amino-4-thiazolyl)-2-(methoxyimino)acetamido]-3-(azidomethyl)-8-oxo-5-thia-1-azabicyclo[4.2.0]oct-2-ene-2-carboxylate.

**5.** A preparation according to claim 2, in which the antibacterial compound is ceftriaxone or a pharmaceutically usable salt, ester or hydrate thereof.

**6.** A preparation according to any one of claims 1-5 in an enteric coated oral dosage form.

**7.** A preparation according to any one of claims 1-5 in a rectal dosage form.

**Revendications**

**1.** Préparation pharmaceutique contenant (a) un composé ayant une activité antibactérienne et (b) une quantité efficace d'un système à 2 composants renforçant la résorption constitué ($b_1$) de polyoxyéthylèneglycollauryléther ayant 12 unités d'oxyde d'éthylène dans la partie polyoxyéthylèneglycol (Laureth 12) et ($b_2$) de caprylate de sodium.

**2.** Préparation selon la revendication 1, dans laquelle le composé ayant une activité antibactérienne est un $\beta$-lactame.

**3.** Préparation selon la revendication 2, dans laquelle le $\beta$-lactame a la formule :

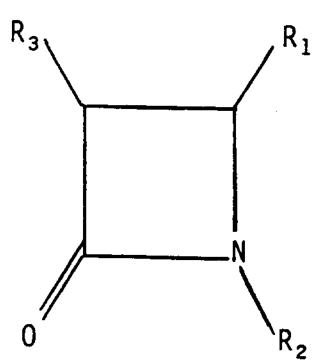

où $R_1$ représente l'hydrogène, un alkyle ou un alkyle substitué, $R_2$ représente $SO_3$-M+, M+ désignant

un proton ou un cation, $R_3$ représente un groupe acylamino ou un hydroxyalkyle ou $R_1$ et $R_2$ représentent ensemble avec le cycle $\beta$-lactame (azétidinone) auquel ils sont liés, le groupe

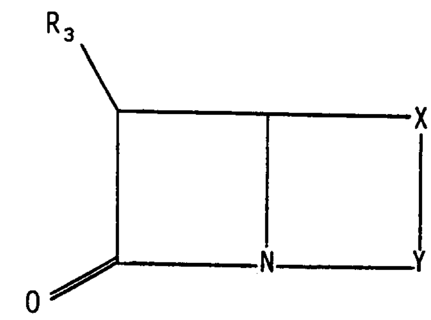

où X est -S-, -O, -SO-, $-SO_2$, $-CH_2$ ou $-CH(CH_3)$ et Y représente un groupe

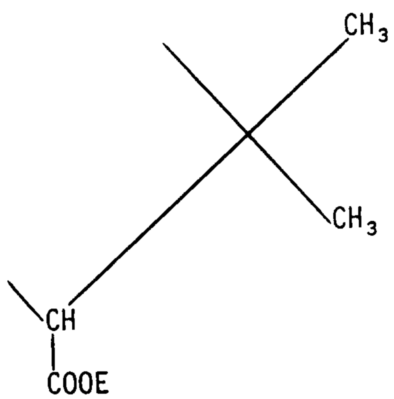

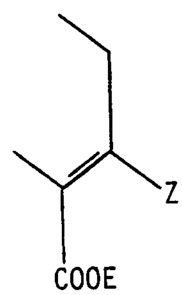

ou

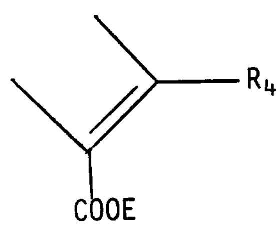

où $R_4$ représente un groupe thio substitué tel que l'éthylthio $-SCH_2CH_2NH_2$,

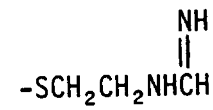

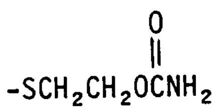

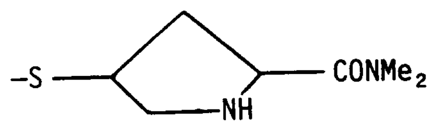

ou un groupe alkyle inférieur facultativement substitué tel que l'aminométhyle, l'acylaminométhyle,

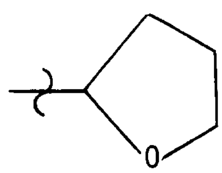

ou un groupe oxy substitué tel que le carbamoyloxy

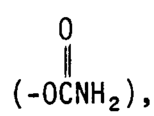

l'atome de C portant le groupe -COOE étant lié à l'atome d'azote du cycle $\beta$-lactame, Z représente l'hydrogène, un halogène, un alcoxy ou $CH_2T$ où T représente l'hydrogène, un alkyle, -CO-O-, le pyridinium, le carboxamidopyridinium, l'aminopyridinium, le carbamoyloxy, l'azido, le cyano, l'hydroxyle, le groupe -S-phényle éventuellement substitué ou représentant le groupe-S-het dans lequel "het" est un hétérocycle à 5 ou 6 chaînons facultativement substitué et E représentant l'hydrogène, un groupe ester pharmaceutiquement utilisable ou un cation salifiable.

**4.** Préparation selon la revendication 2, dans laquelle le composé ayant une activité antibactérienne est le (E)-2-(isobutoxycarbonyl)-2-pentény l-(6R,7R)-7-[(Z)-2-(2-amino-4-thiazolyl)-2-(méthoxyimino)-acétamido]-3-(azidométhyl)-8-oxo-5-thia-1-azabicyclo[4.2.0]oct-2-èn-2-carboxylate.

**5.** Préparation selon la revendication 2, dans laquelle le composé ayant une activité antibactérienne est la Ceftriaxone ou l'un de ses sels, esters ou hydrates pharmaceutiquement utilisables.

**6.** Préparation selon les revendications 1 à 5 sous forme de présentation orale, revêtue d'une couche résistante au suc gastrique.

**7.** Préparation selon les revendications 1 à 5 sous forme de présentation rectale.